# EUROPÄISCHE PATENTSCHRIFT

(11) **EP 3 242 923 B1**
(45) Veröffentlichungstag und Bekanntmachung des Hinweises auf die Patenterteilung: **12.02.2025**
(21) Anmeldenummer: 15816787.4
(22) Anmeldetag: 22.12.2015
(51) Int. Cl.: C10G 35/24

(54) **APPARATUR UND VERFAHREN ZUR UNTERSUCHUNG VON NAPHTHA-REFORMIERUNSPROZESSEN**
APPARATUS AND METHOD FOR STUDYING CATALYTIC REFORMING OF NAPHTHA
APPAREILLAGE ET PROCÉDÉ D'ANALYSE DE REFORMAGE DU NAPHTE

(30) Priorität: 05.01.2015 DE 102015200035
(43) Veröffentlichungstag der Anmeldung: 15.11.2017
(73) Patentinhaber: hte GmbH the high throughput experimentation company, 69123 Heidelberg (DE)
(72) Erfinder: KIRCHMANN, Marius, 69214 Eppelheim (DE); HAUBER, Christoph, 64653 Lorsch (DE)
(74) Vertreter: BASF IP Association
(86) Internationale Anmeldenummer: PCT/EP2015/080911
(87) Internationale Veröffentlichungsnummer: WO 2016/110408

(56) Entgegenhaltungen:
- WO-A1-02/14854
- US-A1- 2013 323 853
- PATRICK KENNEDY J: "New control package is aid to cat reforming", OIL & GAS JOURNAL,, vol. 77, no. 39, 1 September 1979 (1979-09-01), pages 191 - 200, XP001270672
- HENRI L'ONCLE: "automatisation d'une unitÃ CR pilote de reforming catalytique", L'INDUSTRIE DU PETROLE EN EUROPE GAZ-CHIMIE,, vol. 36, no. 388, 1 May 1968 (1968-05-01), pages 54 - 55, XP001270676

## Beschreibung

Die vorliegende Erfindung betrifft ein Verfahren zur Untersuchung von Naphtha-Reformierungsprozessen in Katalysatortestapparaturen mit parallel angeordneten Reaktoren.

Die Naphtha-Reformierung wird zur katalytischen Umsetzung von Rohbenzin (Naphtha) und leichten Ölen eingesetzt, um diese in höherwertige Treibstoffe (Benzin) und Chemikalien (Aromaten) umzuwandeln. Aufgrund des hohen Bedarfs an Treibstoffen und Basischemikalien ist das Naphtha-Reforming von großer wirtschaftlicher und technischer Bedeutung. Weiterhin entsteht beim Naphtha-Reforming Wasserstoff, welcher innerhalb einer Raffinerie für die Hydroprozessierung dringend gebraucht wird. Die Weiterentwicklung und Verbesserung des Naphtha-Reformings ist von Interesse, insbesondere um die Ausbeute an Reformat (Benzin, Aromaten) pro Barrel Naphtha zu steigern und damit wertvolle Ressourcen zu schonen.

Die vorliegende Abhandlung betrifft die Nutzung von Katalysatortestapparaturen zur Durchführung des Naphtha-Reformings im Laborbereich beziehungsweise im Bereich von Pilotanlagen, um die Verfahrensparameter zur Naphtha-Reformings zu verbessern und/oder, um neue Katalysatoren zu entwickeln. Die in Pilotanlagen erhaltenen Untersuchungsergebnisse ermöglichen eine Verbesserung der großtechnischen Prozesse und haben einen hohen Wert für die Industriezweige der Katalysatorherstellung und der petrochemischen Industrie, bei denen die Naphtha-Verarbeitung stattfindet.

Bereits geringfügige Verbesserungen des großtechnischen Naphtha-Reformings haben einen großen Einfluss auf dessen Wirtschaftlichkeit und die damit verbundenen Energiekosten, da die industriellen Dimensionen signifikant sind. Den labortechnischen Methoden unter Verwendung von Katalysatortestapparaturen sind jedoch Grenzen gesetzt, da das Darstellen von großtechnischen Prozessen in Laborapparaturen zahlreichen Fehlerquellen und Schwankungen unterworfen ist. Eine Konsequenz ist, dass gerade geringfügige Verbesserungen aufgrund von Schwankungen der Messgenauigkeit gar nicht und nur mangelhaft erfasst werden können.

Ein besonders wichtiger Parameter, der im Rahmen des Naphtha-Reformings überwacht und/oder optimiert werden muss, ist die sogenannte "Oktanzahl". Die Oktanzahl gibt ein Maß für die Klopffestigkeit von Benzinen an und bildet damit eine Maßzahl für die Qualität von Benzinen. Die Klopffestigkeit von Benzinen wird an Motorentestständen bestimmt und es wird zwischen "RON" (d.h. der Research Octane Number) und der MON (Motor Octane Number) unterschieden. Die Angabe der MON bezieht sich auf die Klopffestigkeit eines Benzins, das bei einer Motorengeschwindigkeit von 900 U/min ermittelt wurde. Die Angabe der RON bezieht sich auf die Klopffestigkeit eines Benzins, dass bei einer Motorengeschwindigkeit von 600 U/min ermittelt wurde. Für die RON- und die MON-Bestimmung an einem Motorenteststand ist jeweils ein halber Liter Benzingemisch erforderlich. In den USA beziehen sich diese Angaben auf den Mittelwerte der Werte, die für RON und MON bestimmt wurden (d.h. die Oktanzahl ergibt sich aus (RON+MON)/2).

Die Ermittlung der Oktanzahl in Laborapparaturen anhand von anderen analytischen Charakterisierungsmethoden während des Reformings, als durch die Bestimmung der Klopffestigkeit von Benzinen an Motorentestständen, ist von großer Bedeutung, um erstens die Herstellung von Proben im Halblitermaßstab zu vermeiden, die sehr aufwendig ist. Zweitens würde dies eine zu geringe Zeitauflösung der Oktanzahl liefern, da diese Proben über einen langen Zeitraum gesammelt werden müssten und damit nur einen Mittelwert über das Zeitintervall liefern würden.

Es ist im Stand der Technik prinzipiell bekannt, wie die Oktanzahl anhand von analytischen Messungen berechnet werden kann, insbesondere durch Gaschromatographie und nahe Infrarotspektroskopie. Zur Ermittlung der Oktanzahl mittels Gaschromatographie wird zunächst die Zusammensetzung des Reformates bestimmt und durch Anwendung von Oktanzahlmodellen die Oktanzahl bestimmt. Für einen Überblick sei vorliegend auf die Veröffentlichung von Knop et al. in Fuel 115 (2014) 666-673 verwiesen, in welcher verschiedene Oktanzahlmodelle vorgestellt und bewertet werden.

Im Stand der Technik sind Verfahren zur Untersuchung des Naphtha-Reformings im Laborbereich prinzipiell bekannt. Bei der Naphtha-Reformierung wird Naphtha mit meist platinhaltigen bifunktionellen Katalysatoren in Kontakt gebracht, bei denen sich feindispergiertes Platin auf (meist chloriertem) Aluminiumoxid befindet. Zudem werden vermehrt bi- oder multimetallische Katalysatoren eingesetzt.

Katalysatorteststände für die Untersuchung von Katalysatoren im Laborbereich ganz allgemein sind im Stand der Technik prinzipiell bekannt. Beispielsweise wird im US-Patent US 7,118,917 B2 von Bergh et al. ein Verfahren und eine Apparatur mit vier oder mehr parallel angeordneten Reaktoren offenbart, bei denen eine individuelle Beheizung der einzelnen Reaktoren möglich ist. Darüber hinaus können die Reaktoren entlang der Reaktorachse mit unterschiedlichen Heizzonen auf unterschiedliche Temperaturen beheizt werden, um dadurch eine genauere Temperaturkontrolle in den Reaktoren zu gewährleisten. Auch sind Reaktionsapparaturen mit parallel angeordneten Reaktoren, die prinzipiell zur Testung von Katalysatoren geeignet sind, bekannt. Als Beispiel hierfür ist die US 2002/0182735 A1 von Chuck Kibby et al. zu nennen, die am 28.05.2002 von der Firma Chevron Texaco Corporation beim Patentamt in den USA eingereicht wurde. In parallel angeordneten Reaktoren können die Produkte hinsichtlich ihrer Eigenschaften untersucht werden, wobei für einen vorbestimmten Produktstrom optimierte Katalysatoren unter Zuhilfenahme kombinatorischer Methoden erhalten werden sollen.

Druckschriften, die im Recherchenbericht genannt werden: WO-A 02/14854 (Chuck Kibby et al.) offenbart ein Verfahren zur Untersuchung von Katalysatoren unter Verwendung von Reaktoren mit Mikrokanälen. Die Reaktoren liegen in der Form von Laminatschichten vor. Der Vorteil ist, dass eine große Anzahl von parallel angeordneten Reaktionskanälen in einer Baugruppe parallel angeordnet werden kann.

In der Veröffentlichung von K.P. Kennedy (Oil and Gas Journal, 492, Vol. 77, No. 39, p. 191 - 200) wird ein Softwarekontrollsystem zur Unterstützung der katalytischen Reformierung beschrieben, das bei großtechnischen Anlagen im Produktionsbetrieb eingesetzt wird. Zahlreiche Betriebsparameter haben einen Einfluss auf die Produktbildung und die damit verbundene Oktanzahl.

In der Veröffentlichung von H. l'Oncle (L'Industrie due Petrole en Europe Gaz-chmie, 36, 388 (1968), p. 54 - 55) wird die Automatisierung einer Pilotanlage zur Durchführung des katalytischen Reformings beschrieben, wobei die Zusammensetzung des Produktstroms analysiert wird und die Regelparameter mittels eines Algorithmus angepasst werden. Hierbei wird auch die Oktanzahl ermittelt.

US-A 2013/0323853 offenbart ein Verfahren zur Überwachung von großtechnischen Reformierungsanlagen unter Verwendung von einer detaillierten Analyse der Kohlenwasserstoffzusammensetzung. Es werden unterschiedliche GC-Methoden zur Analyse der Oktanzahl beschrieben, die miteinander in Beziehung gesetzt werden.

Eine der Aufgaben, die der vorliegenden Erfindung zugrunde liegt, ist es, die Verfahren zur Untersuchung von Katalysatoren in Parallelanlagen speziell im Hinblick auf Naphtha-Reforming Prozesse anzupassen und gegenüber den Verfahren des Standes der Technik zu verbessern. Ein grundlegend zu lösendes technisches Problem ist die fehlende Genauigkeit von Labormethoden, die mit statistischen Fehlern behaftet sind, wobei dadurch die Aussagekraft teilweise stark eingeschränkt ist beziehungsweise keine signifikante Aussagekraft im Labormaßstab möglich ist.

Die hier genannte und zahlreiche weitere Aufgaben, die hier nicht genannt werden, werden durch eine Apparatur und ein Verfahren gelöst, die nachfolgend näher beschrieben werden: Apparatur

Die erfindungsgemäße Apparatur nach Anspruch 1 weist eine Pluralität von parallel angeordneten Reaktoren auf, vorzugsweise rohrförmige Reaktoren, wobei die Apparatur zur Untersuchung des Naphtha-Reformings geeignet ist, wobei die Apparatur weiterhin eine Prozesssteuerung, eine (allen oder einer Vielzahl der Reaktoren gemeinsame) Eduktfluidzufuhr und jeder einzelne Reaktor eine eigene Ausgangsleitung für Produktstrom sowie zumindest eine online-Analyse-Einheit aufweist, wobei weiterhin jeder einzelne Reaktor der Pluralität von Reaktoren mit einem separaten Beheizungssystem ausgestattet ist, wobei die Apparatur dadurch gekennzeichnet ist, dass die Analyse-Einheit mit jeder Ausgangsleitung für Produktfluidstrom in Wirkverbindung steht.

Die Analyse-Einheit arbeitet "online", d.h. es besteht zumindest temporär eine fluidische Verbindung zwischen einem Reaktorausgang und dem Probeneinlass des Analysegeräts. Das bedeutet ferner, dass der Produktfluidstom, oder ein Aliquot von diesem, direkt in ein Analysengerät überführt wird vorzugsweise ist an dieser Stelle kein händisches Manipulieren der Probe erforderlich. Die Analyseeinheit kann die Analyseergebnisse direkt und unmittelbar auswerten und an die Steuereinheit weitergeben. Der Zeitraum zwischen physikalischer Messung des Parameters im Produktfluidstrom und Übergabe der Messdaten an die Steuereinheit beträgt bei einer solchen "online"-Analyse-Einheit vorzugsweise weniger als 100 Millisekunden, weiter vorzugsweise weniger als 10 Millisekunden, weiter vorzugsweise weniger als 1 Millisekunde. Insgesamt sind die Zeiten zur Übergabe der Messdaten an die Steuereinheit sehr kurz.

In einer weiteren Ausführungsform befindet sich in der jeweiligen Ausgangsleitung zweier oder mehrerer Reaktoren und Eingangsseitig des Analyse- Einheit ein Multiport- oder auch Auswahlventil. Dieses Ventil ist dabei so gestaltet, dass gleichzeitig immer nur genau zwischen einem Reaktorabstrom und der Analyse-Einheit eine Wirkverbindung hergestellt wird (dies entspricht dem Multiplexen).

Die Auswahl der Wirkverbindung und damit des Reaktorabstroms wird durch die Prozessteuerung kontrolliert und veranlasst. Die Ergebnisse der Analyseneinheit werden in der Prozesssteuerung derart gespeichert, dass sie sowohl zeitlich als auch wertemäßig dem Reaktor zugeordnet werden können, aus dem das Produktfluid entnommen wurde. Die Veränderung der der variablen Parameter eines Reaktors erfolgt auf der Basis der diesem zugeordneten Daten. (= demultiplexen)

Mittels dieser online-Analyse-Einheit können die Fluidströme aus den einzelnen Ausgangsleitungen sequentiell und / oder parallel analysiert werden.

Die vorliegende Erfindung betrifft eine Apparatur zur Untersuchung von Naphtha-Reforming, wobei die Apparatur aufweist:
eine Pluralität von parallel angeordneten Reaktoren mit Reaktionsräumen (R1, R2,...), die einzelnen Reaktoren weisen ein Katalysatorfassungsvermögen im Bereich von 0,5 - 200 ccm auf,
eine für zwei oder mehr dieser Reaktoren gemeinsame Eduktfluidzufuhr,
eine Prozesssteuerung,
eine Analyse-Einheit

wobei jeder einzelne Reaktor einer individuell regelbaren Beheizungsvorrichtung ausgestattet ist, und eine Ausgangsleitung für Produktfluidstrom aufweist,
dadurch gekennzeichnet, dass die Analyse-Einheit mit jeder dieser Ausgangsleitung für Produktstrom in Wirkverbindung steht, und dass die Analyse-Einheit in funktionalem Zusammenhang mit der Steuerung der Apparatur steht,
wobei die Analyse-Einheit zur Analyse eines gasförmigen Produktfluidstroms geeignet ist, und die Analyse-Einheit gemeinsam mit der Prozessteuerung die Bestimmung und Optimierung einer Oktanzahl in den parallel angeordneten Reaktoren erlaubn, wobei die Analyse-Einheit ein Gas-Chromatograph ist oder die Charakterisierung des gasförmigen Produktfluidstroms mittels IR-Spektroskopie erfolgt, und wobei die Temperatur des jeweiligen Reaktors, aus dessen Ausgangsleitung der Produktfluidstrom analysiert wurde, angepasst werden kann.

### Verfahren

Die erfindungsgemäße Aufgabe wurde weiterhin dahingehend gelöst, dass mittels einer Analyse-Einheit, bevorzugt einer Analyse-Einheit umfassend Gaschromatografie, die Produktzusammensetzung für jeden Reaktor in der Parallelanlage individuell analysiert wird, wobei insbesondere und vorzugsweise das Produktfluid für diese Analyse nicht oder zumindest nicht in einem signifikanten Ausmaße auskondensiert werden muss. Von Bedeutung für das erfindungsgemäße Verfahren ist dabei insbesondere, dass der Transport des zu analysierenden Teils des Produktfluids in der Gasphase erfolgt und dabei keine Auskondensation erfolgt.

Die Erfindung betrifft ein Verfahren nach Anspruch 7, das unter Verwendung der erfindungsgemäßen Apparatur durchgeführt wird, und bei welchem Verfahren in jedem einzelnen Reaktor der Pluralität von Reaktoren R1, R2, ein naphthahaltiger Eduktfluidstrom zugeführt, und im jeweiligen Reaktor mit einem Katalysator bei einer Temperatur im Bereich von 100°C bis 600°C, vorzugsweise im Bereich von 400 bis 550°C, in Kontakt gebracht wird, und zu jeweils einem Produktfluidstrom umgesetzt wird. Jeder einzelne Produktfluidstrom wird über die einzelnen Ausgangsleitungen zu einer Produktfluidaufnahme oder Nachverbrennung geleitet. Die einzelnen Produktfluidströme beziehungsweise Teilströme der einzelnen Ausgangsleitungen werden dabei jeweils den folgenden Verfahrensschritten unterzogen:
(i) eine analytische Charakterisierung mittels einer Analyse-Einheit einschließlich der einzelnen Produktfluidströme, wobei sich die Analyse auf die Quantifizierung von einer oder mehrerer Komponenten, vorzugsweise ein oder mehrere Gruppen von jeweils 10 bis 20 Komponenten, weiter vorzugsweise auf 10 Gruppen von je 1 bis 100 Komponenten bezieht,
(ii) der Berechnung der Oktanzahl und Vergleich der berechneten Oktanzahl mit einem Sollwert,
(iii) Berechnung der Temperaturdifferenz, die zur Konstanthaltung der Oktanzahl notwendig ist
(iv) Anpassung der Temperatur der jeweiligen Beheizungssystems, das den Reaktor umgibt, aus dessen Ausgangsleitung ein Produktstrom analysiert wurde,
zumindest eine Wiederholung der Verfahrensschritte (i) bis (iv) durch Analyse des Produktfluidstroms, der aus den jeweiligen Ausgangsleitungen austritt und Berechnung der jeweiligen Oktanzahl, die den Produktfluidstrom kennzeichnet.

Gemäß einer bevorzugten Ausführungsform betrifft die vorliegende Erfindung ein Verfahren zur Untersuchung von Naphtha-Reforming unter Verwendung einer Apparatur wie vorliegend beschreiben, wobei der naphthahaltige Eduktfluidstrom nachfolgende Komponenten enthält: zumindest ein Kohlenwasserstoff aus der Gruppe mit 5 bis 14 Kohlenstoffatomen, vorzugsweise ein Gemisch von Kohlenwasserstoffen mit 5 bis 14 Kohlenstoffen, sowie Wasserstoff mit einem Wasserstoffgehalt im Bereich von 0,5 - 60 Vol.-%, vorzugsweise 10 - 30 Vol.-%, den einzelnen Reaktoren R1, R2, zugeführt wird, und in den jeweiligen Reaktoren mit einem Katalysator bei einer Temperatur im Bereich von 100°C bis 600°C, vorzugsweise im Bereich von 400°C bis 550°C, in Kontakt gebracht wird,
und zu jeweils einem Produktfluidstrom umgesetzt wird,
und dieser Produktfluidstrom anschließend einer Ausgangsleitung zugeführt wird, wobei
das Verfahren durch folgende Verfahrensschritte charakterisiert ist:
   (i) eine analytische Charakterisierung der einzelnen Produktfluidströme mittels einer Analyse-Einheit, wobei sich die Analyse auf die Quantifizierung von einer oder mehrerer Komponenten, vorzugsweise einer oder mehrere Gruppen von jeweils 1 bis 300 Komponenten, weiter vorzugsweise auf vier Gruppen von je 20 bis 150 Komponenten bezieht,
   (ii) Vergleich des Ergebniswertes der Quantifizierung mit einem Sollwert, wobei der Vergleich die Hinterlegung eines Auswertungsmodells umfasst,
   (iii) Anpassung eines Prozessparameters der sich auf den Reaktionsprozess des jeweiligen Reaktors bezieht, aus dessen Ausgangsleitung der Produktfluidstrom analysiert wurde,
eine Wiederholung der Verfahrensschritte (i) bis (iii) durch Analyse des Produktfluidstroms, der aus den jeweiligen Ausgangsleitungen austritt.

In Bezug auf den Begriff gemeinsame Eduktfluidzufuhr wird auf die Veröffentlichung der PCT-Anmeldung WO 2005/063372 A2 (mit dem Prioritätsdatum 23.12.2003) von der gleichen Anmelderin Bezug genommen. Auch wird in der PCT-Anmeldung WO 2005/063372 A2 offenbart, was unter den Begriffen Druckaufbaugas und Druckhaltegas zu verstehen ist. Darüber hinaus ist die vorliegende Erfindung zwar für Parallelreaktorapparaturen bevorzugt, aber nicht auf Parallelreaktorapparaturen beschränkt, sondern umfasst auch Einzelreaktoren, die gemäß der vorliegenden Offenbarung aufgebaut sind (ohne allerdings zwei oder mehr Reaktoren aufzuweisen). Insbesondere das erfindungsgemäße Verfahren unter Verwendung eines GC und ohne Auskondensieren weist die erfindungsgemäßen Vorteile auch für einen Einzelreaktor auf.

Im Sinne der vorliegenden Erfindung enthält der Produktfluidstrom vorzugsweise weniger als 1% an auskondensiertem Produktfluid, weiter vorzugsweise weniger als 0,5%.

Die Berechnung der Oktanzahl wie für Schritt (ii) notwendig erfolgt anhand geeigneter Oktanzahlmodelle aus der Produktzusammensetzung. Ein software-gestützter Algorithmus übernimmt dabei, unter anderem, die Auswertung der Chromatogramme, bestimmt die Produktzusammensetzung und berechnet die Oktanzahl. Durch die entsprechende im Vorfeld bestimmte Oktanzahl-Temperaturbeziehung des jeweiligen Katalysators wird dann die Temperaturanpassung berechnet. Diese wird dann an die jeweiligen Reaktorheizer als Temperaturdifferenz übermittelt, um die Oktanzahl konstant zu halten.

Das erfindungsgemäße Verfahren umfasst die Verfahrensschritte (i) bis (iii), die eine Regelschleife bilden. Die Durchführung der Verfahrensschritte und die Ausführung der Regelschleife erfolgt für die gesamte Dauer des erfindungsgemäßen Verfahrens. Bevorzugt erfolgt die Ausführung der Regelschleife in Bezug auf jeden einzelnen Reaktor für mindestens 2 bis 10 Durchläufe der Regelschleife. Die Anzahl der Durchläufe der Regelschleife hängt u.a. von der Gesamtdauer der katalytischen Untersuchung ab. Sofern die Katalysatoren für eine Zeit von Wochen und Monaten getestet werden, kann die Anzahl der Durchläufe der Regelschleife entsprechend hoch sein. Im Hinblick auf die Anzahl der Durchläufe ist auch die Deaktivierungsgeschwindigkeit der einzelnen Katalysatoren zu berücksichtigen. Für den Fall von schnell deaktivierenden Katalysatoren ist es bevorzugt, die Regelschleife entsprechend öfter durchlaufen zu lassen, als im Falle von langsam deaktivierenden Katalysatoren.

Bevorzugt ist eine Auswertung von Chromatogrammen, die mit Hilfe von Gaschromatographie erhalten wurden.

Die Auswertung der Chromatogramme umfasst vorzugsweise eine Identifizierung von einzelnen Komponenten beziehungsweise Gruppen von Komponenten. Insbesondere kann durch die Verwendung von GC als Analysemethode, insbesondere im "online"-Betrieb eine hohe Genauigkeit erreicht werden, was von besonderem Vorteil für das erfindungsgemäße Verfahren ist. Vorzugsweise wird bei der Auswertung der Messdaten auf Vergleichsdaten zurückgegriffen, die vorzugsweise im Rahmen von Kalibrierung generiert wurden.

Die online-analytische Charakterisierung kann aufgrund der hohen Komplexität der Multikomponentengemische eine Zeitdauer im Bereich von mehreren Minuten in Anspruch nehmen. Diese Zeitdauer für die Analyse und die Verwendung des bevorzugten Auswertungsmodells hat den Vorteil, dass die Genauigkeit des erfindungsgemäßen Verfahrens gegenüber den Verfahren des Standes der Technik vor allem in Bezug auf die zeitliche Auflösung stark erhöht ist. Die Zeitauflösung für die Untersuchung von Deaktivierungsprozessen ergibt sich auch durch die Zeitdauer für die Analyse der online-analytischen Charakterisierung.

Die analytische Charakterisierung des Produktfluidstroms, vorzugsweise im online-Modus, ist somit der zeitbestimmende Schritt. Die online-analytische Charakterisierung liegt in einem Zeitbereich von 0,1 - 300 min, vorzugsweise in einem Bereich von 1 - 150 min, weiter vorzugsweise in einem Bereich von 10 - 40 min. In einer alternativen Ausführungsform des erfindungsgemäßen Verfahrens erfolgt die Charakterisierung des Produktfluidstroms mittels IR-Spektroskopie. Genau wie bei der GC-Charakterisierung sind entsprechende Kalibrierungsdaten und Methoden in der Steuerung hinterlegt, durch die die Analyse der Komponenten und die Bestimmung der Oktanzahl vorgenommen wird.

Diese Zeitdauer der eigentlichen Analyse ist zu unterscheiden von der Zeitdauer der Übergabe der Daten von Analyse-Einheit an Steuereinheit, welche oben beschreiben wurde und die im Millisekunden-Bereich liegt. Daher ist die Zeitdauer für die Datenübergabe auch nicht der Schritt, der die Geschwindigkeit des erfindungsgemäßen Verfahrens entscheidend beeinflusst.

Zu beachten ist dabei auch, dass eine Vielzahl der eingesetzten Katalysatoren zur Reformierung von naphthahaltigen Eduktströmen in der Regel ausreichend lange Standzeiten und keine sehr schnelle Deaktivierung aufweisen. Somit sind die Zeitdauer für die Durchführung der online-analytischen Charakterisierung und die Zeitabstände für die Wiederholung der Regelschleife ausreichend im Verhältnis zum Deaktivierungsgeschwindigkeit der einzelnen Katalysatoren.

Darüber hinaus kann in einer weiteren Ausführungsform die Versuchsapparatur und das Verfahren jedoch auch noch so konfiguriert werden, dass die Zeitdauer für die Wiederholung der einzelnen Durchläufe der Regelschleife entsprechend angepasst werden kann. Eine Anpassung kann beispielsweise in besonders bevorzugten Ausführungsformen des Verfahrens darin bestehen, dass vorzugsweise ein Mehrkanal-GC eingesetzt wird. Mittels des Mehrkanal-GC können die Produktfluidströme von mehreren Reaktoren gleichzeitig analysiert werden. Darüber hinaus ist es auch bevorzugt, die Apparatur mit mehr als einer online-Analyse-Einheit auszustatten. Besonders bevorzugte Ausführungsformen sind eine Apparatur mit zumindest einer Gruppe von vier parallel angeordneten Reaktoren und einem Zweikanal-GC. Besonders bevorzugt ist eine Apparatur mit acht parallel angeordneten Reaktoren und ein bis zwei Mehrkanal-GC's. Bevorzugt handelt es sich bei den Mehrkanal-GC's um Doppelkanal-GC's, die gleichzeitig die Produktfluidströme von zwei Reaktoren charakterisieren.

Ein weiter bevorzugter Aspekt des erfindungsgemäßen Verfahrens ist die statistischen Auswertung der Daten, wobei diese statistische Auswertung auf einem Auswertemodell basiert, welches vor der Durchführung der katalytischen Testuntersuchungen in der Prozesssteuerung hinterlegt wurde. Das Auswertemodell und der Abgleich zwischen den Ist- und Sollwerten betrifft auch die Plausibilisierung der Daten sowie die Bewertung hinsichtlich des neu einzustellenden Prozessparameters in Form der Reaktortemperatur Das erfindungsgemäße Verfahren wird so durchgeführt, dass der verfahrensentscheidende Prozessparameter jeweils durch die Reaktortemperatur der einzelnen Reaktoren gebildet wird. Dabei ist es im Rahmen der erfindungsgemäßen Vorrichtung von Bedeutung, dass die Reaktoren einzeln beheizt werden können. Eine solche Einzelbeheizung ist für Teststände nicht routinemäßig vorgesehen und auch in den meisten Fällen nicht notwendig und sinnvoll - anders allerdings für die vorliegenden Reforming Verfahren.

In Bezug auf die Eduktfluidzufuhr ist das erfindungsgemäße Verfahren durch eine WHSV von 0,1 bis 50 h⁻¹ und vorzugsweise durch eine WHSV von 0,5 bis 5 h⁻¹ gekennzeichnet.

In Bezug auf die Regelschleife ist es bevorzugt, dass es sich hierbei um eine Ausführungsform mit einem FUZZY-Logik-Regler handelt. Dieser Algorithmus adressiert die Problematik, wie Ausreißer innerhalb des Analysenberichtes behandelt werden. Dabei ist das Auffinden von Peaks und Verwerfen von Peaks eingeschlossen. Der eigentliche Regelalgorithmus stellt letztlich einen Annäherungsalgorithmus dar. Beispiele für einen Annäherungsalgorithmus sind eine Simplex-Näherung, Intervallschachtelung und ähnliche Algorithmen.

Das erfindungsgemäße Verfahren betrifft die katalytische Naphtha-Reformierung im Labormaßstab und bildet die Grundlage für eine Simulation, um im kleinen Maßstab genaue Vorhersagen über das Verhalten von Katalysatoren und Prozessbedingungen des großtechnischen Prozesses zu machen. Prinzipiell sollen die Naphtha-Vorläufe beziehungsweise Edukte durch den katalytischen Prozess einer Veredelung unterzogen werden. Somit ist das erfindungsgemäße Verfahren darauf abgestellt, das Verfahren so zu regeln, dass im Produktfluidstrom über das Vorliegen von bestimmten gewünschten chemischen Komponenten vorbestimmte Zieleigenschaften erreicht werden. Die Produkteigenschaften spielen bei der Auswertung eine entscheidende Rolle und sind ausschlaggebend für die Anpassung der Regelparameter, wobei eine präzise analytische Auswertung erfolgt.

Das Verfahren betrifft die kontinuierliche Erfassung der Eigenschaftswerte der Zielkomponenten und eine geeignete Anpassung der Prozessparameter um ein Erreichen der Zieleigenschaften sicherzustellen.

Die Zieleigenschaft des Produktfluidstroms ist das Erreichen einer vorgegebenen Oktanzahl im Reformat. Vorzugsweise ist das Verfahren in der Art und Weise ausgestaltet, dass die online-analytische Charakterisierung mit einem Modell erfolgt, welches eine hohe Genauigkeit bei der Auswertung liefert.

Das erfindungsgemäße Verfahren ist durch eine hohe Genauigkeit gekennzeichnet. Gegenüber dem Stand der Technik ist die Genauigkeit, die mit dem erfindungsgemäßen Verfahren erreicht werden kann, mit der Genauigkeit von Pilotanlagen vergleichbar, die als Einstranganlagen (also nicht parallel) und in Verbindung mit einer Auskondensation von Produktfluiden durchgeführt werden.

Ein Verfahren zum Betrieb einer großtechnischen Anlage wird beispielhaft in der DE 2224637 beschrieben. Im Verfahren der DE 2224637 wird der Reaktionsausfluss analysiert. Diese Analyse wird zur Bestimmung eines Leistungskennwertes und eines Gesamtumwandlungskennwertes herangezogen. Mittels einer Optimierungseinrichtung wird der Leistungswert optimiert und mittels eines Signals aus der Optimierungseinrichtung und einer Regeleinrichtung wird die Reaktionsschärfe nachgeregelt. Die beispielhafte Ausführungsform ist für eine großtechnische Anlage gezeigt, die mit 100 m³/h Einsatzmaterial beschickt wurde und die 100 m³ Edelmetall-Reformierungskatalysator enthielt. Dabei werden die Produkte in einen bei niedrigen Temperaturen arbeitenden Abscheider überführt.

Die Durchführung von Untersuchungen in (solchen) Pilotanlagen sind jedoch mit erheblich höherem technischem Aufwand verbunden als das erfindungsgemäße Verfahren mittels online-Charakterisierung und ohne Auskondensierung. Diese Genauigkeit ist von entscheidendem Interesse, was den Nutzen der Daten und des Verfahrens angeht, um eine möglichst genaue Aussage über die katalytischen Eigenschaften von Katalysatoren und Verfahren treffen zu können. Dabei ist insbesondere zu berücksichtigen, dass es sich beim Naphtha-Reformierung um ein technisch besonders anspruchsvolles Verfahren handelt, da es die Umsetzung von Multikomponentengemischen betrifft und im Produktfluid eine Vielzahl von Komponenten auftritt. Eine Optimierung des großtechnischen Prozesses ist mit großem Aufwand verbunden, da sich oftmals nur geringfügige Verbesserungen erzielen lassen. Diese geringfügigen Verbesserungen sind jedoch von großem Nutzen.

Ein Vorteil des erfindungsgemäßen Verfahrens ist es auch, dass die analytische Charakterisierung des Produktfluidstroms jeweils direkt anhand der gasförmigen Proben vorgenommen werden kann. Es ist somit nicht erforderlich, die flüssigen Produktkomponenten auszukondensieren bevor die Analyse erfolgt. Hierdurch können bekannte Verfahren zur Testung von Naphtha-Reforming wesentlich vereinfacht werden. Durch den Verzicht auf apparative Baueinheiten, die vorliegend durch den Verzicht auf Kompressor und Kondensor gegeben sind, ist die Konstruktion der erfindungsgemäßen Apparatur mit geringerem technischen Aufwand verbunden. Somit ist das erfindungsgemäße Verfahren vorzugsweise dadurch gekennzeichnet, dass eine online-analytische Charakterisierung des Produktfluids durchgeführt wird, bei dem in zumindest einem der parallel angeordneten Reaktoren keine vorherige Auskondensation des Produktfluids erfolgt.

Durch die apparative Vereinfachung in Verbindung mit dem erfindungsgemäßen Verfahren ist das Verfahren insbesondere auch für die Verwendung in Hochdurchsatzanlagen geeignet, die mit einer Pluralität von parallel angeordneten Parallelreaktoren ausgestattet ist. Es ergibt sich somit ein Synergieeffekt, was die Kombination der technischen Ausgestaltung des erfindungsgemäßen Verfahrens in Verbindung mit Hochdurchsatztestanlagen betrifft, die mit zwei oder mehr parallel angeordneten Reaktoren ausgestattet sind.

Insbesondere ist es in Verbindung mit dem erfindungsgemäßen Verfahren auch bevorzugt, dass es in Hochdurchsatzanlagen eingesetzt wird, die mit vier bis 40 parallel angeordneten Reaktoren ausgestattet sind. Des Weiteren ist es bevorzugt, dass das erfindungsgemäße Verfahren in Verbindung mit Hochdurchsatzanlagen eingesetzt wird, die mit vier bis 20 parallel angeordneten Reaktoren ausgestattet sind.

Derartige Hochdurchsatzanlagen mit parallel angeordneten Reaktoren sind dadurch gekennzeichnet, dass diese im Forschungslabor betrieben werden können, wobei die einzelnen parallel angeordneten Reaktoren durch ein bestimmtes Katalysatorfassungsvermögen charakterisiert sind. Das Katalysatorfassungsvermögen der Reaktoren liegt im Bereich von 0,5 - 200 ccm, vorzugsweise liegt das Katalysatorfassungsvermögen im Bereich von 1 - 100 ccm, weiter vorzugsweise liegt das Katalysatorfassungsvermögen im Bereich von 2 - 50 ccm.

Das erfindungsgemäße Verfahren ist durch eine hohe Langzeitstabilität gekennzeichnet und kann somit beispielsweise über einen Zeitraum von 10 bis 100 Stunden betrieben werden, vorzugsweise wird das Verfahren für einen Zeitraum von 1 Tag bis 180 Tagen betrieben, weiter vorzugsweise wird das Verfahren für einen Zeitraum von 10 bis 90 Tagen betrieben. Die besondere Eignung des erfindungsgemäßen Verfahrens für Langzeituntersuchungen ist von Bedeutung, da es hierdurch möglich ist, die Katalysatoren und das Reforming-Verfahren unter Bedingungen zu testen, die dem großtechnischen Prozess ähneln.

Im Stand der Technik ist es bekannt, vergleichsweise kostspielige und groß dimensionierte Pilotanlagen für die Untersuchung des Naphtha-Reformings einzusetzen. Die Pilotanlagen werden als Einstranganlagen mit größeren Mengen an Katalysatoren betrieben, was zu sehr hohen Betriebskosten führt. Die flüssigen Reaktionsprodukte werden in diesen Pilotanlagen auskondensiert, um dann mittels Motorenteststände die Klopffestigkeit (Oktanzahl) zu ermitteln.

Das Potential des vorliegenden Verfahrens besteht darin, parallel Katalysatoren im Bereich Naphtha Reforming bei konstanter Oktanzahl und somit industrienahen Bedingungen zu testen. Dies kann bei der Entwicklung neuer Katalysatorsysteme, aber auch zum Leistungsvergleich bestehender Katalysatoren einen deutlichen Mehrwert bedeuten.

Für das erfindungsgemäße Verfahren ist es von Bedeutung, dass vorliegend eine online-analytische Charakterisierung durchgeführt wird, wobei diese online-analytische Charakterisierung an einem gasförmigen Produktfluidstrom durchgeführt wird. Insbesondere ist es hierbei auch bevorzugt, dass die online-analytische Charakterisierung des gasförmigen Produktfluidstroms mittels Gaschromatographie durchgeführt wird.

### Naphtha - Zusammensetzung von Eduktstrom und Produktstrom

Das vorliegende Verfahren betrifft die Umsetzung und die Analyse von Multikomponentengemischen, wobei die Gemische unterschiedliche kohlenwasserstoffhaltige Komponenten enthalten. Der Begriff naphthahaltiger Eduktfluidstrom beziehungsweise naphthahaltiger Vorlauf bedeutet ein Gemisch von kohlenwasserstoffhaltigen Komponenten, die 5 bis 14 Kohlenstoffe. Darüber hinaus umfasst der naphthahaltige Eduktfluidstrom auch Wasserstoff, wobei der Wasserstoffanteil des naphthahaltigen Eduktfluidstroms im Bereich von 0,5 - 60 Vol.-%, vorzugsweise 10 - 25 Vol.-% liegt. Die Umsetzung des naphthahaltigen Eduktfluistrom führt zu einem Reformatbenzin, welches einen naphthahaltigen Produktfluidstrom darstellt. Der naphthahaltige Produktfluidstrom kann eine Vielzahl von unterschiedlichen kohlenwasserstoffhaltigen Komponenten aufweisen. Beispielsweise umfasst der naphthahaltige Produktfluidstrom 1 bis 500 Kohlenwasserstoffkomponenten, vorzugsweise umfasst der naphthahaltige Produktfluidstrom 20 bis 400 Kohlenwasserstoffkomponenten, weiter vorzugsweise umfasst der naphthahaltige Produktfluidstrom 20 bis 300 Kohlenwasserstoffkomponenten.

In einer bevorzugten Ausführungsform ist die online-analytische Charakterisierung durch eine sehr hohe Genauigkeit gekennzeichnet, was die qualitative und quantitative Charakterisierung einer Vielzahl von Komponenten betrifft. Hierzu ist auch festzustellen, dass gerade bei der Reformierung von Naphtha eine Vielzahl von unterschiedlichen Produktkomponenten entsteht.

Die Umsetzung des naphthahaltigen Eduktfluidstroms kann bei einem Druck im Bereich von 1,2 - 100 bar durchgeführt werden, vorzugsweise bei einem Druck im Bereich von 1,5 - 100 bar , weiter vorzugsweise wird das erfindungsgemäße Verfahren bei einem Druck im Bereich von 2 - 35 bar durchgeführt.

In einer bevorzugten Ausführungsform des erfindungsgemäßen Verfahrens betrifft das Verfahren eine Steuerung der Katalysatortestungsapparatur zur unter von Katalysatoren bei der Reformierung von Naphtha, das so betrieben wird, dass die Prozessparameter in Verbindung mit den einzelnen Reaktoren so angepasst werden, dass in den Produktfluidströmen aus den einzelnen parallel angeordneten Reaktoren je ein Produktfluidstrom mit einer konstanten Oktanzahl (und zwar der C5+ Fraktion, d.h. der Kohlenwasserstoffe mit 5 oder mehr Kohlenstoffatomen) austritt.

Als Analysengeräte können alle technischen Geräte eingesetzt werden, welche Daten ausgeben, die mit einer Oktanzahl korreliert werden können. Als Referenz gilt, dass iso-Oktan eine Oktanzahl von 100 hat, wohingegen Heptan eine Oktanzahl von 0 aufweist. Die Bedeutung der Oktanzahl besteht darin, das "Klopfverhalten" des Substanzgemisches zu kennzeichnen.

Zu beachten ist, dass das Klopfverhalten und die damit verbundene Oktanzahl für einzelne Komponenten und Gruppen in der Literatur bekannt ist und diese bekannten Daten in das erfindungsgemäße Verfahren einbezogen werden. Anhand der analytischen Charakterisierung und der daraus abgeleiteten Berechnung und dem Vergleich mit hinterlegten Daten kann eine Oktanzahl ermittelt werden. Die Oktanzahl wird vorzugsweise durch gewichtete Summierung der Mengen an einzelnen Komponenten oder Komponentengruppen und deren Klopfeigenschaften gewonnen.

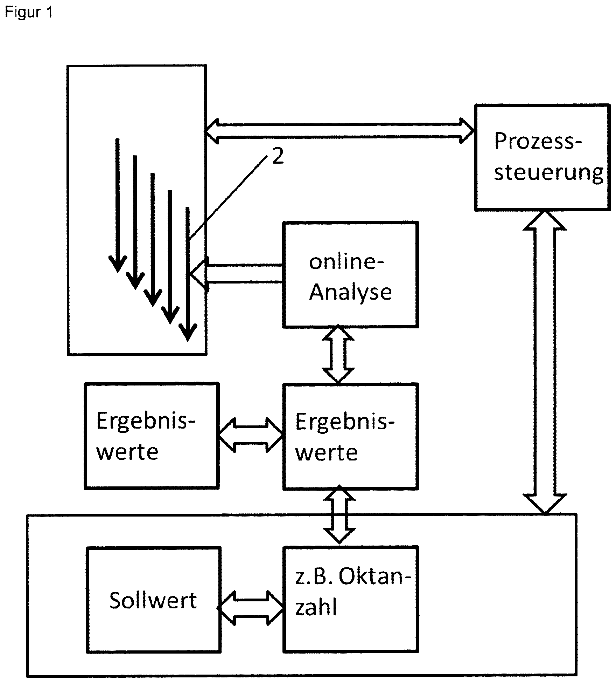

Kurze Beschreibung der Figuren
- Figur 1: zeigt beispielhaft ein Schema des erfindungsgemäßen Verfahrens und der Regelschleife, wobei zunächst eine online-analytische Charakterisierung des Produktfluidstroms von einer Ausgangsleitung erfolgt und zumindest ein Ergebniswert, vorzugsweise eine Vielzahl, mit zumindest einem Sollwert, vorzugsweise einer Vielzahl, abgeglichen werden. Es erfolgt eine Datenanalyse mittels Auswertungsmodell, wobei eine Anpassung von Prozessparametern vorgenommen wird. Es erfolgt eine Wiederholung der Regelschleife. Vorzugsweise wird die Oktanzahl im Produktfluidstrom für die Dauer der katalytischen Testuntersuchung durch eine Anpassung der Temperatur der einzelnen Reaktoren konstant gehalten.

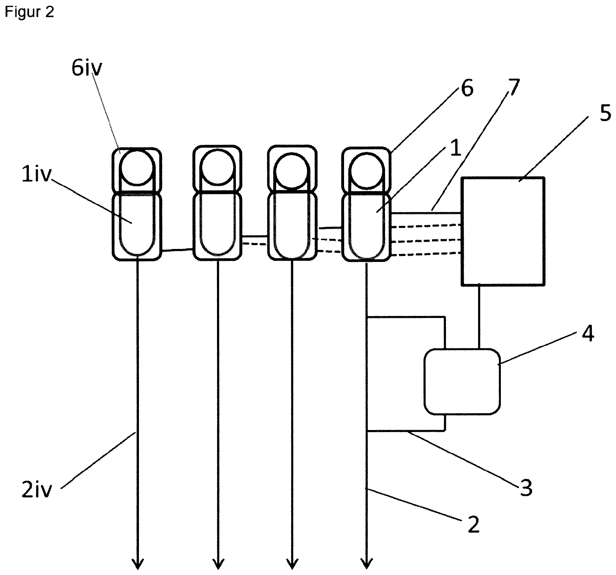

- Figur 2: zeigt schematische Darstellung einer Apparatur mit vier parallel angeordneten Reaktoren, die mit online-Analyse-Einheit ausgestattet sind, bei der sich die Analyse-Einheit in Wirkverbindung mit einer Teilstromleitung von der Ausgangsleitung 1 befindet.
- Figur 3: zeigt im oberen Teil der y-Achse eine schematische Darstellung der Oktanzahlwerte (die konstant gehalten werden während die Temperaturen der unterschiedlichen Katalysatoren in drei parallel angeordneten Reaktoren eingebaut sind, wobei die Messungen in einem Zeitraum von 10 Stunden nachgeführt wurden. Im unteren Teil der y-Ache ist die Nachführung der Temperatur der jeweiligen Reaktorheizung für die jeweiligen Katalysatoren gegeben. Die x-Achse spiegelt diesen Zeitraum wieder, um die Oktanzahl der C5+Kohlenwasserstoffe auf einem konstanten Niveau zu halten.
- Figur 4: zeigt das Schema eines Reglers, der mit einem System gekoppelt ist. "System" bedeutet hierbei Parallelreaktorapparatur. Der Regler erhält zeitabhängige Werte aus den Produktfluidströmen der einzelnen Leitungen und bewirkt danach eine Anpassung eines Prozessparameters.
- Figur 5: zeigt die Übersicht eines Regelkreises, der Bestandteil einer Katalysatortestapparatur ist. Gemäß des erfindungsgemäßen Verfahrens ist der Regelkreis Bestandteil einer Apparatur mit parallel angeordneten Reaktoren.
- Figur 6: zeigt die Übersicht zu einem Regelkreis in einem Verfahrensablauf, wobei hier die Unterteilung zwischen Prozess 1 und Prozess 2 erfolgt, die jeweils zwei unterschiedliche Reaktoreinheiten einer Katalysatortestapparatur bedeuten.

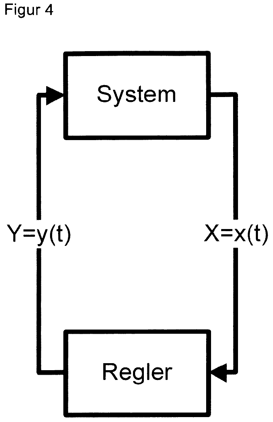

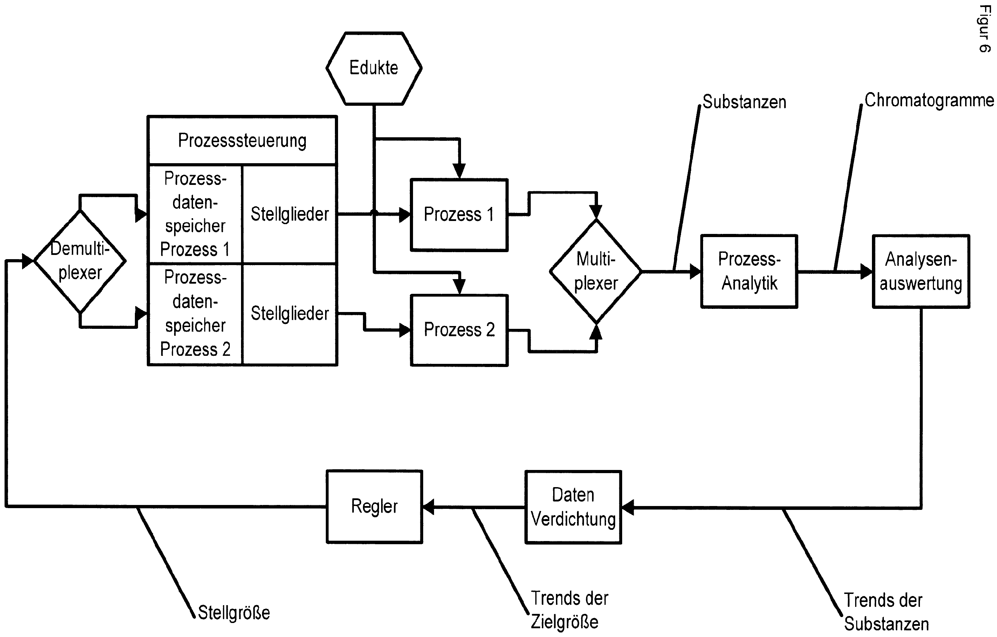

In allen dargestellten Ausführungsformen der Figuren 4 bis 6 bezieht sich die Kopplung der Prozessregelung auf die Verbindung mit einer Parallelreaktorapparatur.

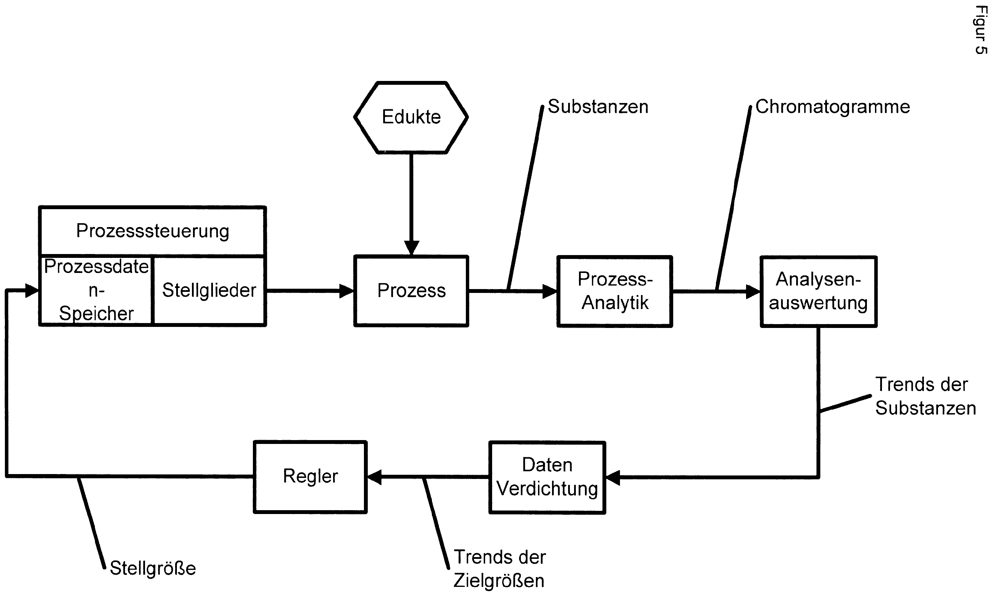

Der Begriff "System" hat vorliegend keine einschränkende Wirkung und betrifft jede Anordnung von Parallelreaktoren. Auch bezieht sich der Begriff "Prozess" in Figur 5 auf das Verfahren in Kombination mit parallel angeordneten Reaktoren.

Die Anzahl der parallel angeordneten Reaktoren ist durch die technische Handhabbarkeit im Hinblick auf Obergrenzen betreffend die Anzahl der Reaktoren begrenzt. Ein Aspekt des erfindungsgemäßen Verfahrens ist die Verwendung von zwei oder mehr parallelen Reaktoren, vorzugsweise von vier oder mehr Reaktoren. Der Parallelisierungsgrad hat einen Einfluss auf die Ausgestaltung des Verfahrens, da die Analysenzeit bei der Analyse von Produktfluiden bestimmten Mindestzeiten unterworfen ist. Neben der Analysenzeit haben auch andere Parameter einen Einfluss auf die Laufzeit. Insbesondere ist es dabei bevorzugt, dass die Analysensäule nach der Durchführung der Analyse regeneriert wird.

Somit ergeben sich Verarbeitungszeiten (Laufzeiten) für die Gruppen Prozessanalytik, Analysenauswertung und Datenverarbeitung. Die Laufzeit in diesen Gruppen beträgt vorzugsweise maximal die Hälfte der Reaktionszeit des Systems (Nyquist-Shannon-Abtasttheorem).

Der Begriff "Multiplexer" bedeutet vorliegend ein Auswahlventil, mit dem die Produktfluidströme der der jeweiligen Reaktoren zeitsequentiell auf das Analysengerät geschaltet wird (welches vorliegend auch als "gemultiplexed" bezeichnet wird). Das "Demultiplexen" des Reglers bedeutet, dass die aus den Daten ermittelte Stellgröße demjenigen Reaktor - und dem in dem Reaktor ablaufenden Prozess - zugeordnet wird, der diese Daten ursprünglich geliefert hat. Daraus wird ersichtlich, dass Multiplexer und Demultiplexer gleich viele Positionen besitzen müssen.

Nachfolgend werden einzelne Verfahrensschritten des erfindungsgemäßen Verfahrens wie in den Figuren 1, 5 und 6 gezeigt näher erläutert:
1. Ein Multikomponentengemisch einer Ausgangsleitung eines Reaktors wird mittels online-Analyse-Einheit in seine Einzelkomponenten aufgetrennt.
2. Die aufgetrennten Einzelkomponenten zeigen mehr oder weniger stark ausgeprägte Unterschiede im Hinblick auf die Retentionszeiten, die mit zeitlichen Trends verbunden sind.
   a. Vorzugsweise steht die analytische Auswertung in einem Zusammenhang mit in einer Gruppierung der Analysendaten nach Siedebereichen und das Ergebnis dieser Auswertung wird vorzugsweise zur Bestimmung der Oktanzahl verwendet. Eine Datenverdichtung auf einen einzelnen Wert ist dabei besonders bevorzugt. Es ist erforderlich, dass dem eigentlichen Regler nur eine zeitabhängige Eingangsgröße zugeführt wird. Weiterhin bevorzugt ist es, dass die Datenverdichtung modellbasiert stattfindet. In Zusammenhang mit dem erfindungsgemäßen Verfahren besteht das Modell aus der Verdichtung der analytischen Charakterisierungsdaten, vorzugsweise bestimmt anhand von online-Gaschromatographie, zu einer Oktanzahl. Darüber hinaus auch denkbar ist, dass die analytische Charakterisierung mittels IR-Spektroskopie erfolgt.
3. Beim Regler handelt es sich um ein Element der Apparatur, welches zeitabhängig auf einen Parameter reagiert.
   a. Im konkreten Fall ist der Regler insbesondere:
      i. programmtechnisch dargestellt (ist ein Algorithmus)
      ii. Ist als Intervall gesteuerter Such- und Näherungsalgorithmus gestaltet.
      iii. Alternative: Simplex Suchalgorithmus, andere Suchalgorithmen sind von der vorliegenden Erfindung auch erfasst.

Durch die Prozessanalytik werden im Fall chromatographischer Methoden zeitdiskret Abbildungen des Prozesses geschaffen. In bestimmten zeitlichen Abständen, der Abtastfrequenz, werden einzelne Proben der Substanzgemische (d.h. der Reformate) entnommen, die aus dem jeweiligen Reaktor austreten.

Die Substanzgemische werden analysiert, d.h. in ihre Komponenten zerlegt. Bei der Naphtha-Reformierung und dem erfindungsgemäßen Verfahren ist es besonders bevorzugt, dass hier eine gaschromatographische Analyse vorgenommen wird. Die gaschromatographische Analyse kann als eine unvollständige Chromatographie angesehen werden, da das Substanzgemisch in Gruppen zerlegt wird, deren gegenseitige Abgrenzung durch charakteristische, bekannte Substanzen erfolgt.

Somit ist es Bestandteil des erfindungsgemäßen Verfahrens, dass mehrere Zeittrends auf einen einzigen Zeittrend komprimiert werden. Vorzugweise tritt der Istwert der Oktanzahl in den Regler ein (und zwar als Funktion x=Oktanzahl(t)). Anstatt des Begriffs Oktanzahl kann auch der Begriff Research Octane Number verwendet werden, der mit RON abgekürzt wird.

Ein Regler im Sinne der vorliegenden Erfindung kann genau eine Eingangsgröße verarbeiten. Daher müssen, sofern das zu regelnde System mehrere Istwerte liefert, diese zu einer einzigen zeitabhängigen Größe verdichtet werden.

Ein Regler kann genau ein System steuern. Die Antwort des Regers (Stellgröße) kann dabei auf mehrere Stellglieder und damit mehrere Einflussmöglichkeiten verteilt werden. So können beispielsweise die Temperatur und der Fluss eines Reaktors an den gleichen Reglerausgang und damit die gleiche Stellgröße geschaltet werden. Das System antwortet dann auf alle Einflussgrößen gleichzeitig.

Bevorzugt werden zwei oder mehr Regler verwendet, die die Temperatur eines Reaktors zu beeinflussen. Es handelt sich dabei bevorzugt um genauso viele Systeme wie Regler. (Thermofühler(1); Regler(2); Stellglied(3); Heizelement(4);Thermofühler(1)). Damit die Regelung respektive die Regelungen funktionieren, muss dafür gesorgt werden, dass die gegenseitige Beeinflussung der Regler ausreichend klein sind. Wird eine bestimmte Schwelle für die gegenseitige Beeinflussung überschritten, kann es zu Schwingung der Systeme kommen. Diese Schwingungen sind in der Regel unerwünscht, da kritische Betriebszustände erreicht werden können.

### Beispiele

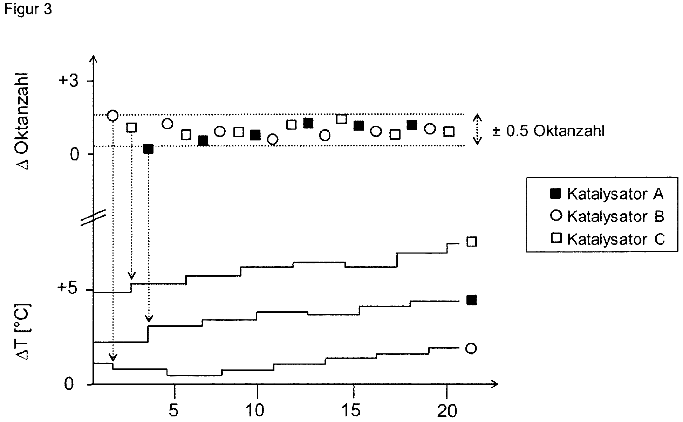

Zur Illustration des erfindungsgemäßen Verfahrens wurde ein Testlauf mit fünf verschiedenen Katalysatoren unter Bedingungen des Naphtha-Reformings durchgeführt. Es wurden industriell hergestellte Katalysatoren eingesetzt und zwar jeweils ca. 9 g Katalysator pro Reaktor. Die Dauer der Untersuchung war 200 Stunden, wobei die Ergebnisse in der Figur 3 ein Ausschnitt aus einem Zeitbereich von 10 Stunden dargestellt sind. Die einzelnen Experimente wurden so durchgeführt, dass die Oktanzahl konstant gehalten wurde, was im oberen Teil der Figur 3 zu erkennen ist. Die Temperatur der einzelnen Reaktoren wurde ständig erhöht, um den Aktivitätsverlust zu kompensieren, der in Verbindung mit der Katalysatoralterung festzustellen war. Im Durchschnitt wurde die Temperatur im Zeitintervall von 200 Stunden jeweils um 1,5 bis 5°C erhöht, um die Oktanzahl des Produktfluidstroms in einem konstanten Bereich zu halten. Die Messwerte für die Oktanzahl zeigen eine Standardabweichung von +/-0,3. Hierbei ist festzustellen, dass es sich hier um eine sehr geringe Standardabweichung handelt, die in dieser Weise mit Laborapparaturen nicht in der Literatur veröffentlicht wurde. Die hohe Genauigkeit ist von großer Signifikanz was die technische Aussagekraft der Daten angeht, die mittels des erfindungsgemäßen Verfahrens erzielt wurden.

### Bezugszeichenliste

- 1: - Reaktor 1
- 1iv: - Reaktor 4
- 2: - Ausgangsleitung in Verbindung mit Reaktor 1
- 2iv: Ausgangsleitung in Verbindung mit Reaktor 4
- 3: Teilstromleitung
- 4: - online-Analyse-Einheit
- 5: Prozesskontrolle
- 6: - Reaktorbeheizung zu Reaktor 1
- 6iv: - Reaktorbeheizung zu Reaktor 4
- 7: - Verbindung von Prozesssteuerung mit Reaktorheizung 1

## Patentansprüche

1. Apparatur zur Untersuchung von Naphtha-Reforming, wobei die Apparatur aufweist:
eine Pluralität von parallel angeordneten Reaktoren mit Reaktionsräumen (R1, R2,···), die einzelnen Reaktoren weisen ein Katalysatorfassungsvermögen im Bereich von 0,5 bis 200 ccm auf,
eine für zwei oder mehr dieser Reaktoren gemeinsame Eduktfluidzufuhr,
eine Prozesssteuerung,
eine Analyse-Einheit,
wobei jeder einzelne Reaktor mit einer individuell regelbaren Beheizungsvorrichtung ausgestattet ist, und eine Ausgangsleitung für Produktfluidstrom aufweist,
**dadurch gekennzeichnet, dass** die Analyse-Einheit mit jeder dieser Ausgangsleitung für Produktstrom in Wirkverbindung steht, und dass die Analyse-Einheit in funktionalem Zusammenhang mit der Steuerung der Apparatur steht,
wobei die Analyse-Einheit zur Analyse eines gasförmigen Produktfluidstroms geeignet ist, und die Analyse-Einheit gemeinsam mit der Prozessteuerung die Bestimmung und Optimierung einer Oktanzahl in den parallel angeordneten Reaktoren erlauben, wobei die Analyse-Einheit ein Gas-Chromatograph ist oder die Charakterisierung des gasförmigen Produktfluidstroms mittels IR-Spektroskopie erfolgt, und wobei die Temperatur der regelbaren Beheizungsvorrichtung des jeweiligen Reaktors, aus dessen Ausgangsleitung der Produktfluidstrom analysiert wurde, angepasst werden kann.

2. Apparatur nach Anspruch 1, die **dadurch gekennzeichnet ist, dass** der Reaktor ein Rohrreaktor ist.

3. Apparatur gemäß einem der vorhergehenden Ansprüche, die **dadurch gekennzeichnet ist, dass** die Apparatur eine Anzahl von 2 bis 40 parallel angeordneten Reaktoren aufweist.

4. Apparatur gemäß einem der vorhergehenden Ansprüche, die **dadurch gekennzeichnet ist, dass** im Innenraum von zumindest einem der parallel angeordneten Reaktoren ein Temperatursensor angeordnet ist.

5. Apparatur gemäß einem der vorhergehenden Ansprüche, die **dadurch gekennzeichnet ist, dass** die Ausgangsleitungen der einzelnen Reaktoren mit einer Druckaufbaugas- und einer Druckhaltegaseinrichtung verbunden sind.

6. Apparatur gemäß einem der vorhergehenden Ansprüche, die **dadurch gekennzeichnet ist, dass** die Ausgangsleitungen der einzelnen Reaktoren mit Membranventilen ausgestattet sind und der Druck innerhalb der Reaktoren durch die Einstellung am Membranventil kontrolliert wird.

7. Verfahren zur Untersuchung von Naphtha-Reforming unter Verwendung einer Apparatur gemäß der Ansprüche 1 bis 6, wobei naphthahaltiger Eduktfluidstrom enthaltend:
ein Gemisch von Kohlenwasserstoffen mit 5 bis 14 Kohlenstoffen, sowie Wasserstoff mit einem Wasserstoffgehalt im Bereich von 0,5 - 60 Vol.-%, den einzelnen Reaktoren R1, R2,... zugeführt wird, und in den jeweiligen Reaktoren mit einem Katalysator bei einer Temperatur im Bereich von 100°C bis 600°C in Kontakt gebracht wird,
und zu jeweils einem Produktfluidstrom umgesetzt wird,
und dieser Produktfluidstrom anschließend einer Ausgangsleitung zugeführt wird, wobei das Verfahren durch folgende Verfahrensschritte charakterisiert ist:
(i) eine analytische Charakterisierung der einzelnen Produktfluidströme mittels einer Analyse-Einheit, wobei sich die Analyse auf die Quantifizierung von einer oder mehrerer Komponenten,
(ii) Vergleichen des Ergebniswertes der Quantifizierung mit einem Sollwert, wobei der Vergleich die Hinterlegung eines Auswertungsmodells umfasst, wobei sich das Auswertungsmodell auf die Berechnung der Oktanzahl bezieht,
(iii) Anpassen der Temperatur der regelbaren Beheizungsvorrichtung des jeweiligen Reaktors, aus dessen Ausgangsleitung der Produktfluidstrom analysiert wurde,
(iv) eine Wiederholung der Verfahrensschritte (i) bis (iii) durch Analyse des Produktfluidstroms, der aus den jeweiligen Ausgangsleitungen austritt.

8. Verfahren zur Untersuchung von Naphtha-Reforming nach Anspruch 7, das **dadurch gekennzeichnet ist, dass** die Zeitdauer für die analytische Charakterisierung in einem Zeitbereich von 0,1 min bis 300 min liegt.

9. Verfahren zur Untersuchung von Naphtha-Reforming nach einem der Ansprüche 7 bis 8, das **dadurch gekennzeichnet ist, dass** das Verfahren über eine Zeitdauer von 24 h bis 3000 h durchgeführt wird.

10. Verfahren zur Untersuchung von Naphtha-Reforming nach einem der Ansprüche 7 bis 9, das **dadurch gekennzeichnet ist, dass** das Verfahren so geführt wird, dass die Anpassung der Oktanzahl so vorgenommen wird, dass die Oktanzahl des Reformats im Produktfluidstrom einen vorgegebenen Zielwert aufweist.

11. Verfahren zur Untersuchung von Naphtha-Reforming nach einem der Ansprüche 7 - 10, das **dadurch gekennzeichnet ist, dass** das Inkontaktbringen des naphthahaltigen Eduktfluidstroms bei einem Druck im Bereich von 1,2 bis 100 bar durchgeführt wird.

## Claims

1. An apparatus for investigating naphtha reforming, the apparatus comprising:
a plurality of reactors arranged in parallel with reaction chambers (R1, R2, ···), the individual reactors having a catalyst capacity in the range from 0.5 to 200 ccm,
a reactant fluid supply common to two or more of these reactors,
a process control,
an analysis unit,
where each individual reactor is equipped with an individually controllable heating device and has an outlet line for product fluid stream,
wherein the analysis unit is operatively connected to each said outlet line for product stream, and wherein the analysis unit is functionally associated with the control of the apparatus,
where the analysis unit is suitable for analyzing a gaseous product fluid stream, and the analysis unit together with the process control allow the determination and optimization of an octane number in the reactors arranged in parallel, where the analysis unit is a gas chromatograph or the gaseous product fluid stream is **characterized by** means of IR spectroscopy, and where the temperature of the controllable heating device of the respective reactor from whose outlet line the product fluid stream was analyzed can be adapted.

2. The apparatus according to claim 1, wherein the reactor is a tubular reactor.

3. The apparatus according to either of the preceding claims, wherein the apparatus has a number of 2 to 40 reactors arranged in parallel.

4. The apparatus according to any of the preceding claims, wherein a temperature sensor is arranged in the interior of at least one of the reactors arranged in parallel.

5. The apparatus according to any of the preceding claims, wherein the outlet lines of the individual reactors are connected to a pressurizing gas unit and a pressure-maintaining gas unit.

6. The apparatus according to any of the preceding claims, wherein the outlet lines of the individual reactors are equipped with membrane valves and the pressure within the reactors is controlled by the setting at the membrane valve.

7. A method for investigating naphtha reforming using an apparatus according to claims 1 to 6, where a naphtha-containing reactant fluid stream comprising: a mixture of hydrocarbons having 5 to 14 carbons, and hydrogen with a hydrogen content in the range of 0.5-60% by volume, is supplied to the individual reactors R1, R2,..., and is contacted in the respective reactors with a catalyst at a temperature in the range from 100°C to 600°C,
and converted to one product fluid stream each,
and this product fluid stream is subsequently supplied to an outlet line, wherein the method is **characterized by** the following method steps:
(i) an analytical characterization of the individual product fluid streams by means of an analysis unit, the analysis relating to the quantification of one or more components,
(ii) comparison of the result value of the quantification with a set value, where the comparison comprises the storage of an evaluation model, where the evaluation model relates to the calculation of the octane number,
(iii) adaptation of the temperature of the controllable heating device of the respective reactor from whose outlet line the product fluid stream was analyzed,
(iv) repetition of method steps (i) through (iii) by analysis of the product fluid stream emerging from the respective outlet lines.

8. The method for investigating naphtha reforming according to claim 7, wherein the duration for the analytical characterization is in a time range from 0.1 min to 300 min.

9. The method for investigating naphtha reforming according to either of claims 7 and 8, wherein the method is carried out over a duration of 24 h to 3000 h.

10. The method for investigating naphtha reforming according to any of claims 7 to 9, wherein the method is conducted such that the octane number is adapted such that the octane number of the reformate in the product fluid stream has a predetermined target value.

11. The method for investigating naphtha reforming according to any of claims 7-10, wherein the contacting of the naphtha-containing reactant fluid stream is carried out at a pressure in the range from 1.2 to 100 bar.

## Revendications

1. Appareil d'analyse du reformage de naphta, l'appareil présentant :
une pluralité de réacteurs agencés en parallèle présentant des espace de réaction (R1, R2, ···), les différents réacteurs présentent un volume dans la plage de 0,5 à 200 cm³,
une alimentation en fluide de départ commune pour deux de ces réacteurs ou plus,
une commande de procédé,
une unité d'analyse,
chaque réacteur individuel étant équipé d'un dispositif de chauffage réglable individuellement et présentant une conduite de sortie pour un flux de fluide produit,
**caractérisé en ce que** l'unité d'analyse est en liaison active avec chaque conduite de sortie pour le flux produit et **en ce que** l'unité d'analyse est en relation fonctionnelle avec la commande de l'appareil,
l'unité d'analyse étant appropriée pour l'analyse d'un flux de fluide produit gazeux et l'unité d'analyse permettant, conjointement avec la commande de procédé, la détermination et l'optimisation d'un indice d'octane dans les réacteurs agencés en parallèle, l'unité d'analyse étant un chromatographe gazeux ou la caractérisation du flux de fluide produit gazeux étant effectuée par spectroscopie IR et la température du dispositif de chauffage réglable de chaque réacteur, à partir de la conduite de sortie duquel le flux de fluide produit a été analysé, pouvant être adaptée.

2. Appareil selon la revendication 1, **caractérisé en ce que** le réacteur est un réacteur tubulaire.

3. Appareil selon l'une des revendications précédentes, **caractérisé en ce que** l'appareil présente un nombre de 2 à 40 réacteurs agencés en parallèle.

4. Appareil selon l'une des revendications précédentes, **caractérisé en ce qu'**un capteur de température est agencé dans l'espace interne d'au moins l'un des réacteurs agencés en parallèle.

5. Appareil selon l'une des revendications précédentes, **caractérisé en ce que** les conduites de sortie des différents réacteurs sont reliées à un dispositif de montée en pression gazeuse et à un dispositif de maintien de pression gazeuse.

6. Appareil selon l'une des revendications précédentes, **caractérisé en ce que** les conduites de sortie des différents réacteurs sont équipées de soupapes à membrane et la pression à l'intérieur des réacteurs est régulée par le réglage au niveau de la soupape à membrane.

7. Procédé d'analyse du reformage de naphta à l'aide d'un appareil selon les revendications 1 à 6, un flux produit de départ contenant du naphta, contenant : un mélange d'hydrocarbures comprenant 5 à 14 atomes de carbone ainsi que de l'hydrogène présentant une teneur en hydrogène dans la plage de 0,5 - 60% en volume, étant introduit dans les différents réacteurs R1, R2,... et étant mis en contact dans les différents réacteurs avec un catalyseur à une température dans la plage de 100°C à 600°C,
et étant transformé à chaque fois en un flux de fluide produit,
et ce flux de fluide produit étant ensuite introduit dans une conduite de sortie, le procédé étant **caractérisé par** les étapes de procédé suivantes :
(i) caractérisation analytique des différents flux de fluide produit au moyen d'une unité d'analyse, l'analyse se rapportant à la quantification d'un ou de plusieurs composants,
(ii) comparaison du résultat de la quantification à une valeur de consigne, la comparaison comprenant le dépôt d'un modèle d'évaluation, le modèle d'évaluation se rapportant au calcul de l'indice d'octane,
(iii) adaptation de la température du dispositif de chauffage réglable du réacteur respectif à partir de la conduite de sortie duquel le flux de fluide produit a été analysé,
(iv) répétition des étapes de procédé (i) à (iii) par analyse du flux de fluide produit qui sort des différentes conduites de sortie.

8. Procédé d'analyse du reformage de naphta selon la revendication 7, **caractérisé en ce que** la durée pour la caractérisation analytique se situe dans une plage temporelle de 0,1 min à 300 min.

9. Procédé d'analyse du reformage de naphta selon l'une des revendications 7 à 8, **caractérisé en ce que** le procédé est réalisé sur une durée de 24 h à 3000 h.

10. Procédé d'analyse du reformage de naphta selon l'une des revendications 7 à 9, **caractérisé en ce que** le procédé est dirigé de manière telle que l'adaptation de l'indice d'octane est mise en œuvre de telle sorte que l'indice d'octane du reformat dans le flux de fluide produit présente une valeur cible prédéfinie.

11. Procédé d'analyse du reformage de naphta selon l'une des revendications 7 à 10, **caractérisé en ce que** la mise en contact du flux de fluide de départ contenant du naphta est réalisé à une pression dans la plage de 1,2 à 100 bars.
